# EUROPÄISCHE PATENTSCHRIFT

(11) **EP 1 274 354 B1**
(45) Veröffentlichungstag und Bekanntmachung des Hinweises auf die Patenterteilung: **27.09.2006**
(21) Anmeldenummer: 00915082.2
(22) Anmeldetag: 19.04.2000
(51) Int. Cl.: A61B 17/60, A61B 17/64, A61B 17/80

(54) **VORRICHTUNG ZUR GELENKARTIGEN VERBINDUNG ZWEIER KÖRPER**
DEVICE FOR THE ARTICULATED CONNECTION OF TWO BODIES
DISPOSITIF PERMETTANT LA CONNEXION ARTICULEE DE DEUX CORPS

(43) Veröffentlichungstag der Anmeldung: 15.01.2003
(73) Patentinhaber: Synthes AG Chur, 7002 Chur (CH)
(72) Erfinder: SCHLÄPFER, Fridolin, CH-8750 Glarus (CH); HESS, Martin, CH-4434 Hölstein (CH)
(74) Vertreter: Lusuardi, Werther
(86) Internationale Anmeldenummer: PCT/CH2000/000224
(87) Internationale Veröffentlichungsnummer: WO 2001/078613

(56) Entgegenhaltungen:
- WO-A-00/69351
- WO-A-94/00066
- US-A- 5 047 029
- US-A- 5 607 426

## Beschreibung

Die Erfindung bezieht sich auf eine Vorrichtung zur gelenkartigen Verbindung zweier Körper.

Bei verschiedenen chirurgischen Implantaten, wie beispielsweise mehrteiligen und gelenkig verbundenen Knochenplatten und osteosynthetischen Fixationsvorrichtungen im Wirbelsäulenbereich, wo beispielsweise ein ungefähr parallel zur Wirbelsäule gerichteter Längsträger mit einer Anzahl Knochenschrauben verbunden werden muss, müssen die gelenkartigen Verbindungen lösbar blockierbar sein. Zu diesem Zweck werden elastische Spannelemente, wie beispielsweise Spannzangen eingesetzt. Die Blockierung solcher gelenkartiger Verbindungen erfolgt durch Aufweiten dieser elastischen Spannelement innerhalb eines korrespondierenden festen Hohlraumes. Dieser Aufweitvorgang von elastischen Spannelementen wird bevorzugt durch Klemmkonusse oder -keile erreicht.

Eine osteosynthetische Fixationsvorrichtung mit einer in einem Knochen zu verankernden Knochenschraube, einem Längsträger und einem Verbindungselement, mittels welchem die Knochenschraube am Längsträger fixierbar ist, ist aus der WO 94/00066 SCHLÄPFER bekannt. Diese bekannte Fixationsvorrichtung umfasst ein elastisches, kugelschichtförmiges Klemmelement, welches eine konische Bohrung aufweist, und eine Knochenschraube, welche einen zur konischen Bohrung des Klemmelementes korrespondierenden Aussenkonus und vom Knochen entfernt, endständig ein Gewinde aufweist. Im Verbindungselement ist zur Aufnahme des Klemmelementes ein sphärischer Hohlraum vorgesehen, dessen Abmessungen so gestaltet sind, dass die Kugelschicht des Klemmelementes darin schwenkbar aufnehmbar ist. Eine zweite Bohrung dient zur Aufnahme des Längsträgers. Beim Anziehen einer Mutter, welche über das endständige Gewinde der Knochenschraube geschraubt wird und auf dem Klemmelement aufsteht, wird der Konus an der Knochenschraube in den Innenkonus des Klemmelementes gezogen, wodurch dieses gespreizt und gegen die Wandung des sphärischen Hohlraumes gepresst wird und somit das Verbindungselement und die Knochenschraube relativ zueinander fixiert werden.

Nachteilig bei allen diesen Konusklemmverbindungen ist das schwierige Lösen einer solchen Verbindung, was oft nur durch starke Hammerschläge auf die Knochenschraube oder das Verbindungselement oder durch Spezialinstrumente möglich ist.

Hier will die Erfindung Abhilfe schaffen. Der Erfindung liegt die Aufgabe zugrunde, eine Klemmverbindung basierend auf einem Konus- respektive Keilmechanismus herzustellen, welche mittels eines Schraubendrehers feststellbar und auch wieder lösbar ist.

Aus der WO 00/69351, welche einen Stand der Technik gemäß Art. 54(3)(4) bildet, ist eine Vorrichtung zur Verbindung zweier Körper bekannt, welche ein die zwei Körper verbindendes lösbar blockierbares Gelenk umfasst. Das Gelenk ist mittels einer spreizbaren Büchse blockierbar, wobei die Büchse mittels eines durch eine Schraube bewegbaren Klemmkonusses lösbar spreizbar ist.

Die Erfindung löst die gestellte Aufgabe mit einer Vorrichtung zur gelenkartigen Verbindung zweier Körper, welche die Merkmale des Anspruchs 1 aufweist.

Die erfindungsgemässe Vorrichtung zur gelenkartigen Verbindung zweier Körper umfasst einen ersten Körper mit einem zu einer Zentralachse koaxialen, die Gelenkschale beinhaltenden Hohlraum, ein Klemmelement mit einer Längsachse und einer koaxialen Bohrung, wobei das Klemmelement zum Hohlraum komplementär und senkrecht zur Längsachse elastisch deformierbar ausgestaltet ist, mindestens ein zur Längsachse koaxial in die Bohrung einführbares und axial sich erweiterndes Spannelement, so dass durch axiales Verschieben des mindestens einen Spannelementes in der Bohrung das Klemmelement expandierbar und im Hohlraum lösbar fixierbar ist, einen zweiten Körper, welcher mittels des mindestens einen Spannelementes und des Klemmelementes mit dem ersten Körper lösbar verbindbar ist und ein Antriebselement, mittels welchem das mindestens eine Spannelement axial relativ zur Bohrung verschiebbar ist. Das Antriebselement ist sowohl mit dem Klemmelement als auch mit dem mindestens einen Spannelement um die Längsachse rotierbar und axial formschlüssig verbunden.

### Version A:

In einer nicht der erfindungsgemässen Vorrichtung ist die um die Längsachse rotierbare und axial formschlüssige Verbindung zwischen dem Antriebeselement und dem Klemmelement mittels eines ringförmigen Absatzes am Antriebselement und einer dazu komplementären ringförmigen Nute im Klemmelement realisiert, während die um die Längsachse rotierbare und axial formschlüssige Verbindung zwischen dem Antriebselement und dem Spannelement durch eine Gewindeverbindung realisiert ist.

### Version B:

In einer anderen nicht erfindungsgemässen Vorrichtung sind die Ausgestaltungen der um die Längsachse rotierbaren und axial formschlüssigen Verbindungen gegenüber der bevorzugten Ausführungsform vertauscht. Die um die Längsachse rotierbare und axial formschlüssige Verbindung zwischen dem Antriebeselement und dem Klemmelement ist durch eine Gewindeverbindung realisiert, während die um die Längsachse rotierbare und axial formschlüssige Verbindung zwischen dem Antriebselement und dem Spannelement mittels eines ringförmigen Absatzes am Antriebselement und einer dazu komplementären ringförmigen Nute im Spannelement realisiert ist.

### Version C:

In einer erfindungsgemässen Vorrichtung sind beide der um die Längsachse rotierbaren und axial formschlüssigen Verbindungen, diejenige zwischen Antriebselement und Klemmelement, sowie diejenige zwischen Antriebselement und Spannelement durch Gewindeverbindungen ausgestaltet. Eine der beiden Gewindeverbindungen ist mit linksgängigen Gewinden und die andere der beiden Gewindeverbindungen mit rechtsgängigen Gewinden ausgestaltet. Dabei können die Gewindesteigungen der beiden Gewindeverbindungen den gleichen Betrag oder einen ungleichen Betrag aufweisen.

Die Vorteile der Versionen A und B liegen in deren einfacher Handhabung, während die Version C dank der beiden Gewindeverbindungen ein schnelles Lösen und Anziehen gestattet und zudem einfach herzustellen ist.

Die für die Art der Gelenkverbindung massgebende Ausgestaltung des Hohlraumes im ersten Körper und des Klemmelementes kann je nach Anwendung der erfindungsgemässen Vorrichtung zylindrisch oder sphärisch, vorzugsweise kugelschichtförmig sein. Bei einer zylindrischen Ausgestaltung ist das Klemmelement nur um die Zentralachse des Hohlraumes rotierbar, während bei einer kugelschichtförmigen Ausgestaltung des Hohlraumes das Klemmelement um die Zentralachse sowie um zwei weitere, senkrecht dazu stehende Achsen rotierbar ist.

In wiederum einer Ausführungsform der erfindungsgemässen Vorrichtung ist der zweite Körper einstückig mit dem Spannelement. Diese Ausführungsform eignet sich speziell für die Anwendung der erfindungsgemässen Vorrichtung innerhalb eines Wirbelsäulenfixationssystemes, wobei der zweite Körper vorzugsweise als Knochenschraube und der erste Körper als Verbindungselement zwischen der Knochenschraube und einem Längsträger ausgebildet ist. Hierzu eignet sich die Ausgestaltung der Gelenkverbindung als Kugelgelenkverbindung mit kugelschichtförmigem Hohlraum.

In einer anderen Ausführungsform der erfindungsgemässen Vorrichtung ist der erste Körper als Knochenplatte ausgebildet, während der zweite Körper ein Verbindungselement zu einer weiteren Knochenplatte bildet, so dass insgesamt eine Knochenfixationsvorrichtung mit zwei gelenkig verbundenen Knochenplatten gebildet wird. Hierzu eignet sich eine bezüglich nur einer Achse schwenkbare Ausgestaltung der Gelenkverbindung mit kreiszylindrischem Hohlraum.

Die erfindungsgemässe Vorrichtung kann in verschiedenen Ausführungsformen auch mehrere Spannelemente umfassen, welche mittels des Antriebselementes koaxial zur Längsachse und relativ zur Bohrung in beide Richtungen verschiebbar sind. Bei Ausführungsformen mit zwei Spannelementen weist das Klemmelement vorzugsweise zwei an seine Enden angrenzende elastisch deformierbare Längenabschnitte auf, so dass je ein Spannelement von einem Ende her in die Bohrung einführbar ist.

Weitere vorteilhafte Ausgestaltungen der Erfindung sind in den abhängigen Ansprüchen gekennzeichnet.

Die durch die Erfindung erreichten Vorteile sind im wesentlichen darin zu sehen, dass dank der erfindungsgemässen Vorrichtung eine Klemmverbindung basierend auf einem Konus- respektive Keilmechanismus mittels eines Schraubendrehers und ohne weitere Hilfsmittel feststellbar und auch wieder lösbar ist.

Die Erfindung und Weiterbildungen der Erfindung werden im folgenden anhand der teilweise schematischen Darstellungen mehrerer Ausführungsbeispiele noch näher erläutert.

Es zeigen:
Fig. 1 einen Schnitt durch eine nicht erfindungsgemässe Vorrichtung zur Verbindung eines Knochenfixationselementes mit einem weiteren Implantat;
Fig. 2 einen Schnitt durch eine weitere nicht erfindungsgemässe Vorrichtung zur Verbindung eines Knochenfixationselementes mit einem weiteren Implantat;
Fig. 3 einen Schnitt durch wiederum eine erfindungsgemässe Vorrichtung zur Verbindung eines Knochenfixationselementes mit einem weiteren Implantat;
Fig. 4 eine perspektivische Darstellung einer Ausführungsform der erfindungsgemässen Vorrichtung zur Verbindung zweier plattenförmiger Körper;
Fig. 5 ein Detail eines der Körper der in Fig. 4 dargestellten Ausführungsform der erfindungsgemässen Vorrichtung;
Fig. 6 einen Schnitt durch eine weitere Ausführungsform der erfindungsgemässen Vorrichtung;
Fig. 7 eine Ansicht einer Anwendung der in Fig. 6 dargestellten Ausführungsform der erfindungsgemässen Vorrichtung an einer Wirbelsäule; und
Fig. 8 einen Schnitt durch eine Anwendung der in Fig. 3 dargestellten Ausführungsform der erfindungsgemässen Vorrichtung innerhalb eines Wirbelsäulen-fixationssystemes.

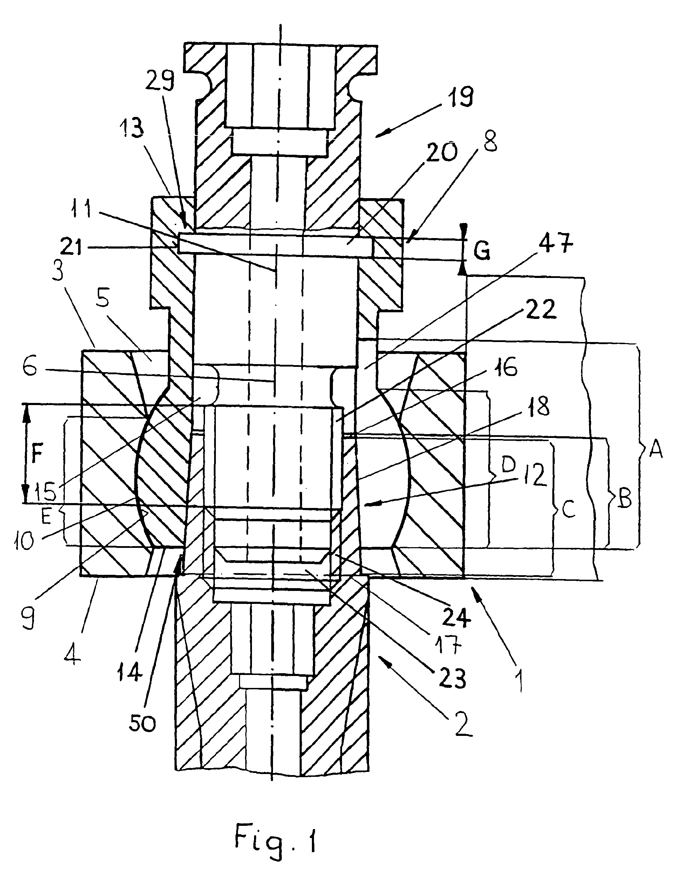

Fig. 1 zeigt eine nicht erfindungsgemässe Vorrichtung, welche einen als Verbindungselement ausgebildeten ersten Körper 1 mit einer Oberseite 3, einer zur Oberseite 3 parallelen Unterseite 4 und einem Hohlraum 5 mit einer zur Oberseite 3 senkrecht stehenden Zentralachse 6, einen als Pedikelschraube ausgebildeten zweiten Körper 2, ein mit dem zweiten Körper 2 einstückiges Spannelement 12, ein Klemmelement 8 und ein als Spannschraube ausgebildetes Antriebselement 19 umfasst Der Hohlraum 5 durchdringt den ersten Körper 1 koaxial zur Zentralachse 6 und enthält einen axialen, bezüglich der Zentralachse 6 rotationssymmetrischen Längenabschnitt E (N = 1), welcher sphärisch konkav ausgebildet ist. Korrespondierend zur sphärisch konkaven Form des Längenabschnittes E umfasst das Klemmelement 8 einen Längenabschnitt D (N = 1), so dass das Klemmelement 8 um die Zentralachse 6 sowie um zwei weitere, zur Zentralachse 6 senkrecht stehende und des Zentrum der sphärischen Form schneidende Achsen im Hohlraum 5 rotierbar ist. Das Klemmelement 8 umfasst daneben eine Längsachse 11, ein oberes Ende 13, ein unteres Ende 14 und koaxial zur Längsachse 11 eine Bohrung 15 und eine Öffnung 50, welche axial aneinander grenzen, so dass das Klemmelement 8 zwischen dem oberen Ende 13 und den unteren Ende 14 durchdrungen wird. Diese Öffnung 50 verjüngt sich konisch vom unteren Ende 14 her auf einem Längenabschnitt B (N = 1) und ist auf einem diesen Längenabschnitt B enthaltenden Längenabschnitt A (N = 1) elastisch und senkrecht zur Längsachse 11 deformierbar. Die elastische Deformierbarkeit wird durch Schlitze 47 hergestellt, wobei vorzugsweise vier von der Unterseite 14 parallel zur Längsachse 11 eindringende Schlitze 47 um 90° auf dem Umfang versetzt angeordnet sind. Das Spannelement 12 (M = 1) ist in dieser Ausführungsform einstückig mit dem zweiten Körper 2 ausgeführt, wobei das untere Ende 17 an den zweiten Körper 2 angrenzt. Das Spannelement 12 wird mit seinem oberen Ende 16 vom unteren Ende 14 her in die Öffnung 50 im Klemmelement 8 eingeführt. Das Spannelement 12 verjüngt sich axial zum oberen Ende 16 hin auf einem Längenabschnitt C (N = 1) komplementär konisch zum Längenabschnitt B der Öffnung 50, so dass das Spannelement 12 und das Klemmelement 8 eine konische Klemmverbindung 18 bilden. Durch koaxiale Verschiebung des Spannelementes 12 in der Öffnung 50 wird der elastische Längenabschnitt A radial gespreizt oder kann bei Verschiebung des Spannelementes 12 in die andere Richtung in seine Ausgangslage zurückfedern. Diese konische Klemmverbindung gestattet ein lösbares Fixieren des mit dem Spannelement 12 verbundenen zweiten Körpers 2 mit dem ersten Körper 1. Die axiale Verschiebung des Spannelementes 12 erfolgt durch Drehen des als Spannschraube ausgebildeten Antriebselementes 19 um die Längsachse 11. Die rotierbare und axial formschlüssige Verbindung 29 zwischen dem Antriebselement 19 und dem Klemmelement 8 auf dem Längenabschnitt G ist durch einen koaxialen ringförmigen Absatz 20 am Klemmelement 8 und eine entsprechende ringförmige Nute 21 in der Bohrung 15 ausgestaltet. Die ebenfalls um die Längsachse 11 rotierbare und axial formschlüssige Verbindung zwischen dem Antriebselement 19 und dem Spannelement 12 ist durch eine Gewindeverbindung mit einem Aussengewinde 22 am Antriebselement 19 und einem dazu korrespondierenden Innengewinde 24 in einer Bohrung 23, welche vom oberen Ende 16 des Spannelementes 12 in dieses eindringt, ausgeführt. Mittels dieser Gewindeverbindung ist das Spannelement 12 durch Drehen des Antriebselementes 19 innerhalb des Klemmelementes 8 axial verschiebbar, während die Axialkräfte zwischen Antriebselement 19 und Klemmelement 8 durch den in der Nute 21 gelagerten Absatz 20 am Antriebselement 8 aufgenommen werden.

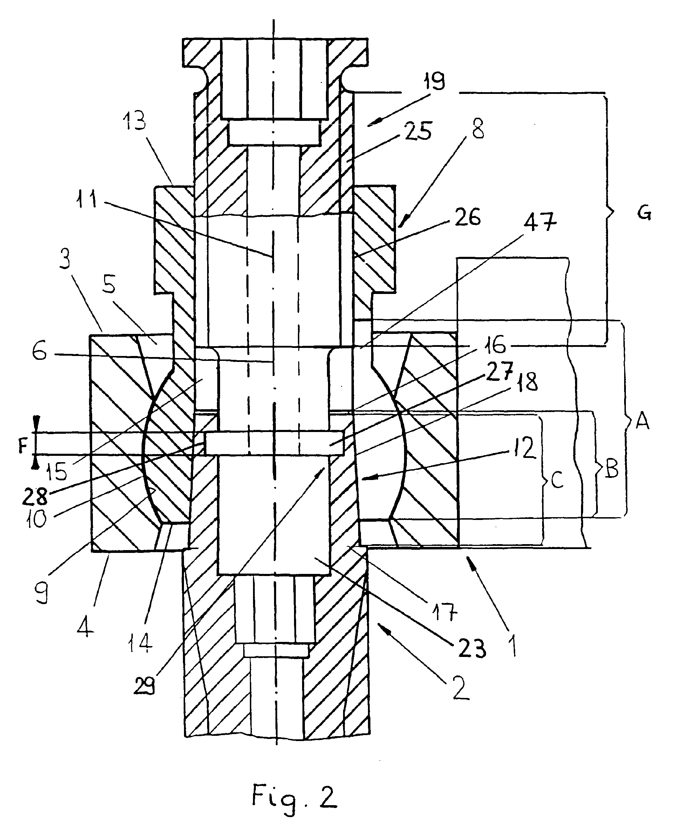

Fig. 2 zeigt eine nicht erfindungsgemässe Vorrichtung, welche sich von der in Fig. 1 dargestellten Ausführungsform Vorrichtung nur darin unterscheidet, dass die axiale Verschiebung des Spannelementes 12 im Klemmelement 8 durch eine Gewindeverbindung, welche ein Aussengewinde 25 auf dem Längenabschnitt G am Antriebselement 19 und ein dazu komplementäres Innengewinde 26 in der Bohrung 15 umfasst, erfolgt. Die um die Längsachse 11 rotierbare und axial formschlüssige Verbindung zwischen dem Antriebselement 19 und dem Spannelement 12 erfolgt über Verankerungsmittel 29, welche auf dem Längenabschnitt F des Antriebselementes 19 angebracht sind und die Axialkräfte zwischen dem Antriebselement 19 und dem Spannelement 12 aufnehmen. Die Ausgestaltung dieser Verankerungsmittel 29 erfolgt durch einen koaxialen ringförmigen Absatz 27 am Antriebselement 19, welcher in einer ringförmigen Nute 28 gelagert ist, wobei diese Nute 28 in einer vom oberen Ende 16 des Spannelementes 12 koaxial in dieses eindringenden Bohrung 23 angebracht ist.

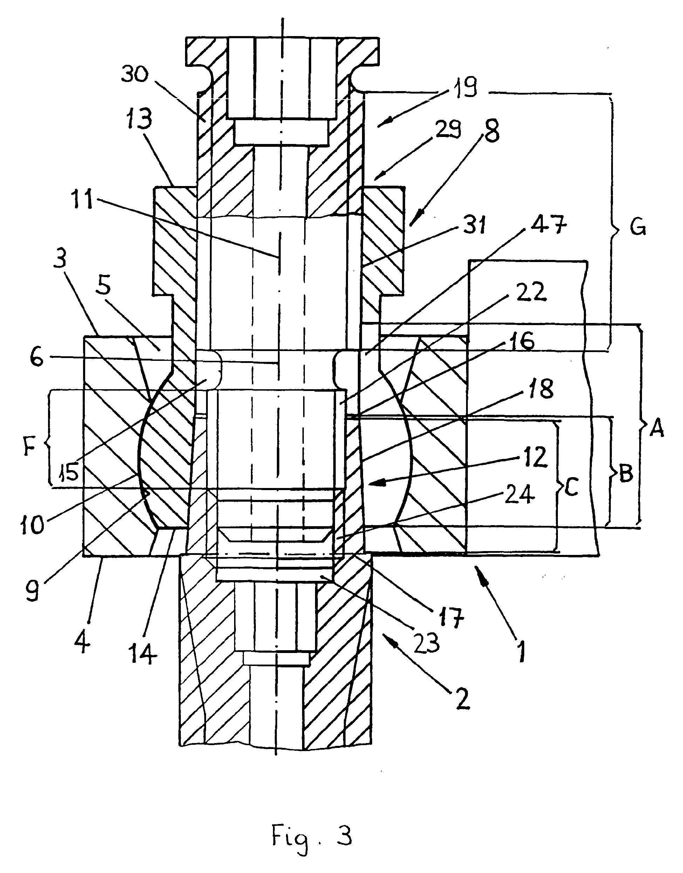

Fig. 3 zeigt eine Ausführungsform der erfindungsgemässen Vorrichtung, welche eine Kombination der in den Fig. 1 und 2 dargestellten Ausführungsformen darstellt. Das Antriebselement 19 ist einerseits mittels einer ersten Gewindeverbindung, welche ein Aussengewinde 22 auf dem Längenabschnitt F des Antriebselementes 19 und ein dazu komplementäres Innengewinde 24 im Spannelement 12, welches in einer konzentrisch zur Längsachse 11 vom oberen Ende 16 in das Spannelement 12 eindringenden Bohrung 23 angebracht ist, umfasst. Andererseits ist das Antriebselement 19 mittels einer zweiten Gewindeverbindung, welche ein Aussengewinde 30 auf dem Längenabschnitt G des Antriebselementes 19 und ein dazu komplementäres Innengewinde 31 in der Bohrung 15 des Klemmelementes 8 umfasst, mit dem Klemmelement 8 um die Längsachse 11 rotierbar und axial formschlüssig verbunden.

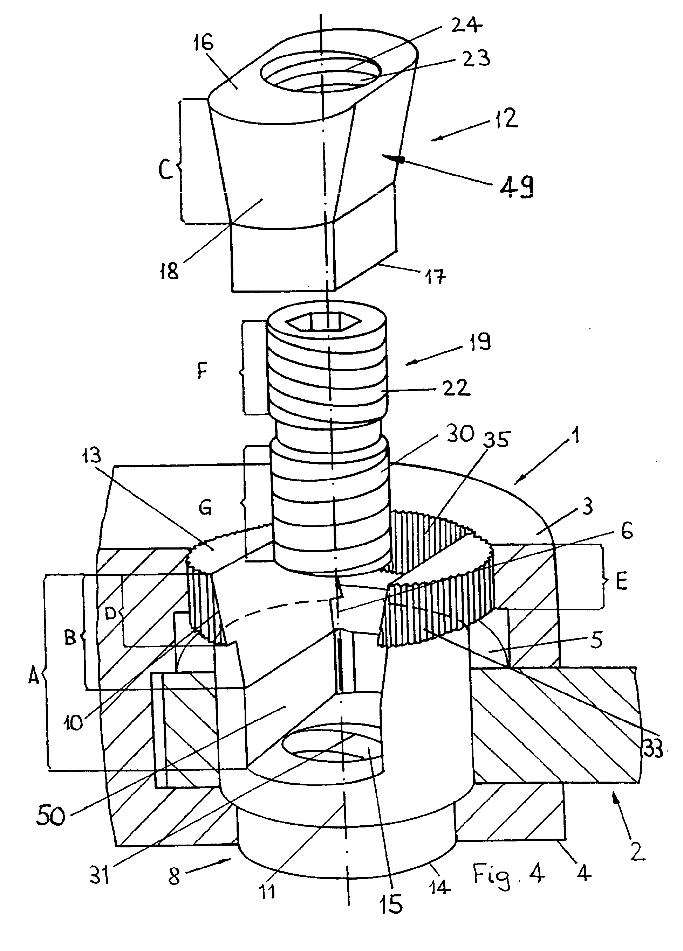

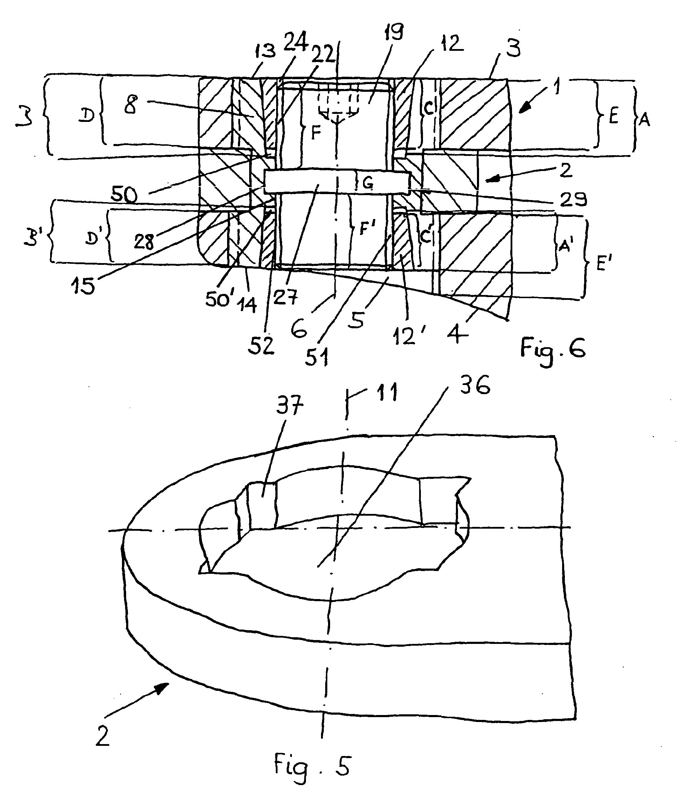

Fig. 4 zeigt eine Ausführungsform der erfindungsgemässen Vorrichtung, welche einen ersten plattenförmigen Körper 1 mit einer Oberseite 3, einer zur Oberseite 3 parallelen Unterseite 4 und einem Hohlraum 5 mit einer zur Oberseite 3 senkrecht stehenden Zentralachse 6, einen zweiten plattenförmigen Körper 2, ein Spannelement 12, ein Klemmelement 8 und ein als Spannschraube ausgebildetes Antriebselement 19 umfasst. Das Klemmelement 8 bildet die Drehachse der gelenkartigen Verbindung. Der Hohlraum 5 durchdringt den ersten Körper 1 koaxial zur Zentralachse 6 und enthält einen bezüglich der Zentralachse 6 rotationssymmetrischen Längenabschnitt E (N = 1), welcher kreiszylindrisch ausgebildet ist. Korrespondierend zur kreiszylindrischen Form des Längenabschnittes E umfasst das Klemmelement 8 einen Längenabschnitt D (N = 1), so dass das Klemmelement 8 um die Zentralachse 6 im Hohlraum 5 rotierbar ist. Das Klemmelement 8 umfasst daneben eine Längsachse 11, ein oberes Ende 13, ein unteres Ende 14 und koaxial zur Längsachse 11 eine Bohrung 15 und eine Öffnung 50, welche axial aneinander grenzen, so dass das Klemmelement 8 zwischen dem oberen Ende 13 und den unteren Ende 14 durchdrungen wird. Die Öffnung 50 verjüngt sich keilförmig vom oberen Ende 13 her auf einem Längenabschnitt B (N = 1) und ist auf einem diesen Längenabschnitt B enthaltenden Längenabschnitt A (N = 1) elastisch und senkrecht zur Längsachse 11 deformierbar. Die elastische Deformierbarkeit wird dadurch erreicht, dass die Öffnung 50 das Klemmelement 8 auf dem Längenabschnitt A auch senkrecht zur Längsachse 11 durchdringt. Das Spannelement 12 wird mit seinem unteren Ende 17 vom oberen Ende 13 her in die Öffnung 50 im Klemmelement 8 eingeführt. Das Spannelement 12 verjüngt sich parallel zur Längsachse 11 auf einem Längenabschnitt C (N = 1) komplementär zum Längenabschnitt B der Öffnung 50, so dass das Spannelement 12 und das Klemmelement 8 eine keilartige Klemmverbindung 49 bilden. Durch koaxiale Verschiebung des Spannelementes 12 in der Öffnung 50 wird der elastische Längenabschnitt A radial gespreizt oder kann durch Verschieben des Spannelementes 12 in die andere Richtung in seine Ausgangslage zurückfedern. Vom unteren Ende 17 bis zum Längenabschnitt C ist das Spannelement 12 vierkantig und zum Innenvierkant 37 (Fig. 5) in der zur Längsachse 11 koaxialen Bohrung 36 im zweiten Körper 2 korrespondierend ausgestaltet, was eine Fixierung des zweiten Körpers 2 mit dem Spannelement 12 bezüglich Rotation um die Längsachse 11 gestattet. Die Verschiebung des Spannelementes 12 parallel zur Längsachse 11 erfolgt durch Drehen des als Spannschraube ausgebildeten Antriebselementes 19 um die Längsachse 11. Das Antriebselement 19 ist einerseits mittels einer ersten Gewindeverbindung, welche ein Aussengewinde 22 auf dem Längenabschnitt F des Antriebselementes 19 und ein dazu komplementäres Innengewinde 24 im Spannelement 12 umfasst, mit dem Spannelement 12 um die Längsachse 11 rotierbar und axial formschlüssig verbunden, wobei das Innengewinde 24 in einer konzentrisch zur Längsachse 11 das Spannelement 12 durchdringenden Bohrung 23 angebracht ist,. Andererseits ist das Antriebselement 19 mittels einer zweiten Gewindeverbindung, welche ein Aussengewinde 30 auf dem Längenabschnitt G des Antriebselementes 19 und ein dazu komplementäres Innengewinde 31 in der Bohrung 15 des Klemmelementes 8 umfasst, mit dem Klemmelement 8 um die Längsachse 11 rotierbar und axial formschlüssig verbunden. Zur Sicherung gegen Rotation um die Zentralachse 6 ist das Klemmelement 8 auf dem Längenabschnitt D mit einer Aussenverzahnung 33 ausgestattet, welche mit einer dazu korrespondierenden Innenverzahnung 35 auf dem Längenabschnitt E des Hohlraumes 5 in Eingriff bringbar ist.

In Fig. 6 ist eine Ausführungsform der erfindungsgemässen Vorrichtung dargestellt, welche sich von der in Fig. 4 gezeigten Ausführungsform nur dadurch unterscheidet, dass sie ein Klemmelement 8 mit einer ersten Öffnung 50, welche vom oberen Ende 13 her einen ersten konisch oder keilförmig sich verjüngenden Längenabschnitt B aufweist, und einer zweiten Öffnung 50', welche vom unteren Ende 14 her einen zweiten konisch oder keilförmig sich verjüngenden Längenabschnitt B' aufweist, sowie zwei dazu komplementäre Spannelemente 12; 12' umfasst. Das Antriebselement 19 ist mittels Verankerungsmittel 29 um die Zentralachse 6 rotierbar und axial formschlüssig im Klemmelement 8 gelagert. Die Verankerungsmittel 29 bestehen analog zu der in Fig. 2 beschriebenen Ausführungsform aus einem konzentrischen ringförmigen Absatz 27 auf dem Längenabschnitt G am Antriebselement 19, welcher in einer dazu komplementären ringförmigen Nute 28 im Klemmelement 8 gelagert ist. Beide Spannelemente 12;12' sind mittels Gewindeverbindungen mit dem Antriebselement 19 verbunden, wobei eine der Gewindeverbindungen eine rechtsgängige Gewindeverbindung und die andere Gewindeverbindung eine linksgängige Gewindeverbindung ist.

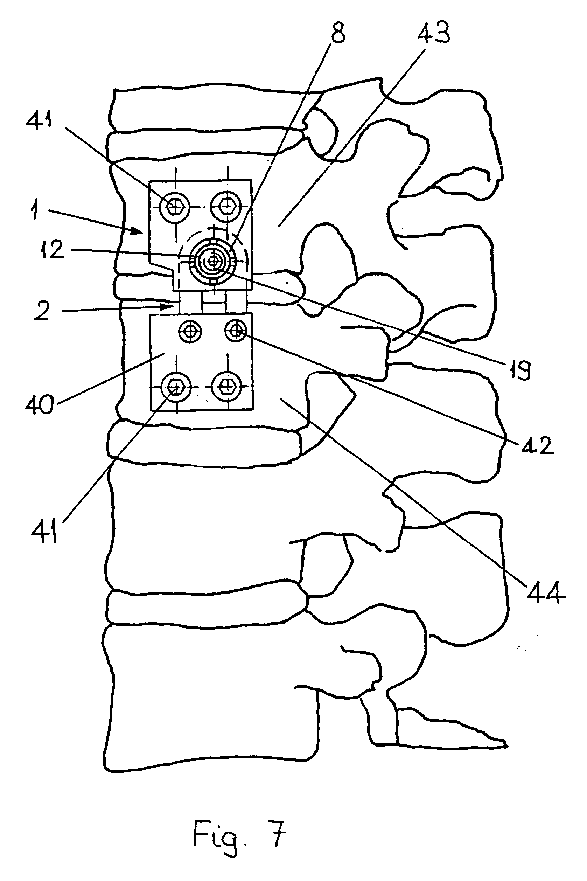

Fig. 7 zeigt die Anwendung der in Fig. 6 dargestellten Ausführungsform der erfindungsgemässen Vorrichtung als mehrteilige Knochenplatte, welche lateral an einer Wirbelsäule befestigt ist. Der Körper 1 bildet einen am kranialen Wirbelkörper 43 mittels Knochenschrauben 41 befestigten ersten Teil der Knochenplatte, während der Körper 2 als Verbindungselement zu einem zweiten, am kaudalen Wirbelkörper 44 befestigten Teil 40 der Knochenplatte dient. Mittels des Klemmelementes 8, welches im Körper 1 um die senkrecht zur Oberfläche des Wirbelkörpers 43 stehende Zentralachse 6 (Fig. 6) rotierbar gelagert ist, ist der Körper 2 einseitig gelenkartig mit dem Körper 1 verbunden. Der zweite Teil 40 der Knochenplatte ist an der anderen Seite des Körpers 2 parallel zur Wirbelsäulenlängsachse verschiebbar und mittels zweier Stellschrauben 42 fixierbar, so dass die Knochenplatte eine winklige Bewegung der Wirbelkörper 43;44 relativ zueinander und eine Verschiebung der Wirbelkörper parallel zur Wirbelsäulenlängsachse zulässt. Das Gelenk ist mittels des Antriebselementes 19 feststellbar.

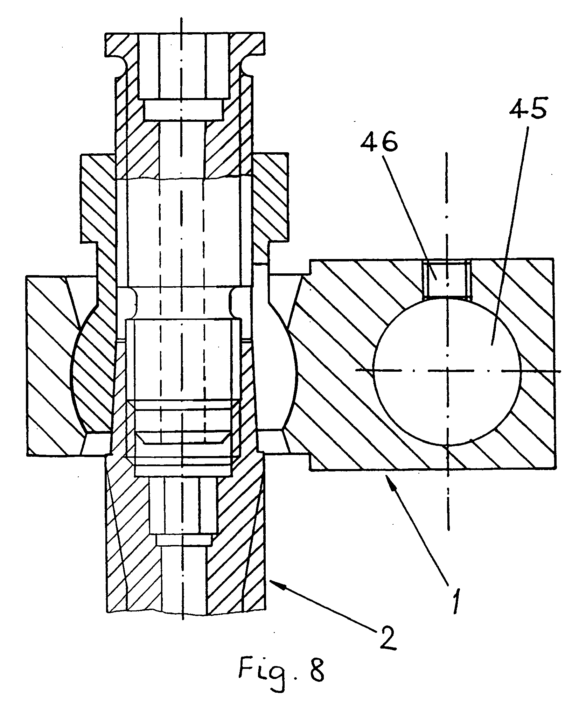

Fig. 8 zeigt die Anwendung der in Fig. 3 dargestellten Ausführungsform der erfindungsgemässen Vorrichtung als Verbindungselement innerhalb eines Wirbelsäulenfixationssystems. Der Körper 1 dient als Verbindungselement zwischen dem als Pedikelschraube ausgebildeten Körper 2 und einem Längsträger 45. Der Längsträger 45 wird beispielsweise mittels einer Schraube 46 im Körper 1 fixiert. Ein solches Wirbelsäulenfixationssystem ist beispielsweise aus der WO 94/00066 SCHLÄPFER et al. bekannt.

## Patentansprüche

1. Vorrichtung zur gelenkartigen Verbindung zweier Körper, mit
A) einem ersten Körper (1), welcher eine Oberseite (3), eine Unterseite (4) und einen zu einer Zentralachse (6) koaxialen Hohlraum (5) umfasst, wobei die Zentralachse (6) die Oberseite (3) und die Unterseite (4) schneidet und der Hohlraum (5) mindestens in die Oberseite (3) mündet;
B) einem Klemmelement (8) mit einer Längsachse (11), einem oberen Ende (13), einem unteren Ende (14) und einer zur Längsachse (11) koaxialen Bohrung (15), welche in mindestens eine Öffnung (50) mündet, wobei die Aussenkontur des Klemmelementes (8) im Bereich der mindestens einen Öffnung (50) zum Hohlraum (5) komplementär und senkrecht zur Längsachse (11) elastisch deformierbar ausgestaltet ist;
C) mindestens einem zur Längsachse (11) koaxial in die Öffnung (50) einführbaren Spannelement (12), so dass durch axiales Verschieben des mindestens einen Spannelementes (12) in der entsprechenden Öffnung (50) das Klemmelement (8) expandierbar und im Hohlraum (5) lösbar fixierbar Ist;
D) einem zweiten Körper (2), welcher mittels des mindestens einen Spannelementes (12) und des Klemmelementes (8) mit dem ersten Körper (1) lösbar verbindbar ist;
E) einem Antriebselement (19), mittels welchem das mindestens eine Spannelement (12) axial relativ zur entsprechenden Öffnung (50) verschiebbar ist, wobei
F) das Antriebselement (19) sowohl mit dem Klemmelement (8) als auch mit dem mindestens einen Spannelement (12) um die Längsachse (11) rotierbar und axial formschlüssig verbunden ist, wobei
G) das Antriebselement (19) mittels einer ersten Gewindeverbindung, welche ein Aussengewinde (22) auf einem ersten Längenabschnitt (F) und ein dazu komplementäres Innengewinde (24) im Spannelement (12) umfasst. mit dem Spannelement (12) verbunden ist ; und wobei
H) das Antriebselement (19) mittels einer zweiten genenläufigen Gewindeverbindung, welche ein Aussengewinde (30) auf einem zweiten Längenabschnitt (G) und ein dazu komplementäres Innengewinde (31) in der Bohrung (15) des Klemmelementes (8) umfasst, mit dem Klemmelement (8) verbunden ist.

2. Vorrichtung nach Anspruch 1, **dadurch gekennzeichnet, dass** das Klemmelement (8) entlang der Längsachse (11) durchgängig offen ist.

3. Vorrichtung nach Anspruch 1 oder 2, **dadurch gekennzeichnet, dass** das Klemmelement (8) X Schlitze (47) umfasst, welche im wesentlichen axial verlaufen und die Öffnung (50) mit der Aussenkontur des Klemmelementes (8) verbinden.

4. Vorrichtung nach Anspruch 3, **dadurch gekennzeichnet, dass** X = 1 ist.

5. Vorrichtung nach Anspruch 3, **dadurch gekennzeichnet, dass** X = 2 ist und die beiden Schlitze (47) kollateral gegenüber liegen.

6. Vorrichtung nach Anspruch 3, **dadurch gekennzeichnet, dass** X ≤ 12 und X ≥ 3, vorzugsweise X = 6 ist.

7. Vorrichtung nach Anspruch 5, **dadurch gekennzeichnet, dass** die beiden Schlitze (47) und die entsprechende Öffnung (50) nahtlos ineinander übergehen.

8. Vorrichtung nach einem der Ansprüche 3 bis 7, **dadurch gekennzeichnet, dass** die mindestens eine Öffnung (50) koaxial zur Längsachse (11) liegt.

9. Vorrichtung nach Anspruch 8, **dadurch gekennzeichnet, dass** die mindestens eine Öffnung (50) rotationssymmetrisch ist.

10. Vorrichtung nach einem der Ansprüche 1 bis 9, **dadurch gekennzeichnet, dass** die Längsachse (11) und die Zentralachse (6) fluchten.

11. Vorrichtung nach einem der Ansprüche 1 bis 10, **dadurch gekennzeichnet, dass** die Aussenkontur des Klemmelementes (8) mindestens im Kontaktbereich mit der, den Hohlraum (5) begrenzenden Wandung zur Längsachse (11) rotationssymmetrisch ist.

12. Vorrichtung nach einem der Ansprüche 1 bis 11, **dadurch gekennzeichnet, dass** sie mindestens ein Spannelement (12) umfasst, wobei dieses mindestens eine Spannelement (12) mittels des Antriebselementes (19) koaxial zur Längsachse (11) und relativ zu der mindestens einen Öffnung (50) in beide Richtungen verschiebbar ist.

13. Vorrichtung nach einem der Ansprüche 1 bis 12, **dadurch gekennzeichnet, dass**
A) die Wandung des Hohlraumes (5) axial unter anderem N (N ≥ 1) bezüglich der Zentralachse (6) rotationssymmetrische Längenabschnitte (Eᵢ) (i = 1 bis N) umfasst;
B) das Klemmelement (8) axial mindestens K (K ≥ 1) Längenabschnitte (Aᵢ) (i = 1 bis K) aufweist, welche senkrecht zur Längsachse (11) elastisch deformierbar sind;
C) die Aussenkontur des Klemmelementes (8) im Bereich der Längenabschnitte(Aᵢ) (i = 1 bis K) axial mindestens N (N ≥ 1) zur den Längenabschnitten (Eᵢ) (i = 1 bis N) des Hohlraumes (5) in Geometrie und Oberflächenstruktur komplementäre Längenabschnitte (Dᵢ) (i = 1 bis N) aufweist;
D) die Vorrichtung M (M ≥ 1) Spannelemente (12) mit je einem oberen Ende (16) und einem unteren Ende (17) umfasst;
E) die mindestens eine Öffnung (50) des Klemmelementes (8) axial insgesamt mindestens M (M ≥ 1), mit den M (M ≥ 1) Spannelementen (12) beim Spreizen in Kontakt kommende Längenabschnitte (Bᵢ) (i = 1 bis M) aufweist;
F) die M (M ≥ 1) Spannelemente (12) mittels des Antriebselementes (19) koaxial in der mindestens einen Öffnung (50) in beiden Richtungen aktiv verschiebbar sind und damit die elastischen Längenabschnitte (Aᵢ) (i = 1 bis K) senkrecht zur Längsachse (11) gespreizt beziehungsweise zum Zusammengehen gebracht werden und somit die Längenabschnitte (Dᵢ) (i = 1 bis N) in den Längenabschnitten (Eᵢ) (i = 1 bis N) des Hohlraumes (5) blockierbar respektive lösbar sind; wobei
G) das Antriebselement (19) auf einem Längenabschnitt (G) koaxial zur Längsachse (11) mit dem Klemmelement (8) und auf M (M ≥ 1) Längenabschnitten (Fⱼ) (j = 1 bis M) koaxial zur Längsachse (11) mit den M (M ≥ 1) Spannelementen (12) um die Längsachse (11) rotierbar und axial formschlüssig verbunden ist.

14. Vorrichtung nach Anspruch 13, **dadurch gekennzeichnet, dass** die mindestens eine Öffnung (50) axial sich mindestens im Bereich der Längenabschnitte (Dᵢ) (i = 1 bis N) verjüngt.

15. Vorrichtung nach Anspruch 13, **dadurch gekennzeichnet, dass** die Spannelemente (12) axial sich mindestens im Bereich der Längenabschnitte (Cᵢ) (i = 1 bis M) verjüngen.

16. Vorrichtung nach Anspruch 14, **dadurch gekennzeichnet, dass** die Spannelemente (12) axial sich im Bereich der Längenabschnitte (Cᵢ) (i = 1 bis M) komplementär zu den Längenabschnitten (Dᵢ) (i = 1 bis N) verjüngen.

17. Vorrichtung nach einem der Ansprüche 13 bis 16, **dadurch gekennzeichnet, dass** K = N ist.

18. Vorrichtung nach einem der Ansprüche 13 bis 17, **dadurch gekennzeichnet, dass** K = M ist.

19. Vorrichtung nach einem der Ansprüche 13 bis 18, **dadurch gekennzeichnet, dass** M = N ist.

20. Vorrichtung nach einem der Ansprüche 13 bis 19, **dadurch gekennzeichnet, dass** K = 1 ist.

21. Vorrichtung nach einem der Ansprüche 13 bis 20, **dadurch gekennzeichnet, dass** M = 1 ist.

22. Vorrichtung nach einem der Ansprüche 13 bis 21, **dadurch gekennzeichnet, dass** N = 1 ist.

23. Vorrichtung nach den Ansprüchen 20 bis 22, **dadurch gekennzeichnet, dass** das Spannelement (12) konzentrisch zur Längsachse (11) eine Bohrung (23) aufweist und das Antriebselement (19) auf dem Längenabschnitt (Fᵢ) (i = 1) koaxial zur Längsachse (11) in die Bohrung (23) schraubbar ist und auf dem Längenabschnitt (G) durch Verankerungsmittel (2) mit dem Klemmelement (8) um die Längsachse (11) rotierbar und axial formschlüssig verbunden ist.

24. Vorrichtung nach Anspruch 23, **dadurch gekennzeichnet, dass** der Steigungswinkel der beiden Gewindeverbindungen identisch ist.

25. Vorrichtung nach Anspruch 22 oder 23, **dadurch gekennzeichnet, dass** der Steigungswinkel der beiden Gewindeverbindungen ungleich ist.

26. Vorrichtung nach einem der Ansprüche 13 bis 25, **dadurch gekennzeichnet, dass** das Klemmelement (8) im Bereich der Längenabschnitte (Eᵢ) (i = 1 bis N) des Hohlraumes (5) kraftschlüssig mit dem ersten Körper (1) verbindbar ist.

27. Vorrichtung nach einem der Ansprüche 13 bis 25, **dadurch gekennzeichnet, dass** das Klemmelement (8) im Bereich der Längenabschnitte (Eᵢ) (i = 1 bis N) des Hohlraumes (5) formschlüssig mit dem ersten Körper (1) verbindbar ist.

28. Vorrichtung nach einem der Ansprüche 13 bis 27, **dadurch gekennzeichnet, dass** die M (M ≥ 1) Spannelemente (12) sich auf den Längenabschnitten (Cᵢ) (i = 1 bis M) konisch verjüngen.

29. Vorrichtung nach einem der Ansprüche 13 bis 27, **dadurch gekennzeichnet, dass** das Klemmelement (8) sich auf den Längenabschnitten (Bᵢ) (i = 1 bis M) konisch verjüngt.

30. Vorrichtung nach den Ansprüchen 28 und 29, **dadurch gekennzeichnet, dass** die Verjüngungen komplementär sind.

31. Vorrichtung nach einem der Ansprüche 13 bis 27, **dadurch gekennzeichnet, dass** die M (M ≥ 1) Spannelemente (12) sich auf den Längenabschnitten (Cᵢ) (i = 1 bis M) keilförmig verjüngen.

32. Vorrichtung nach einem der Ansprüche 13 bis 27, **dadurch gekennzeichnet, dass** das Klemmelement (8) sich auf den Längenabschnitten (Bᵢ) (i = 1 bis M) keilförmig verjüngt.

33. Vorrichtung nach den Ansprüchen 31 und 32, **dadurch gekennzeichnet, dass** die Verjüngungen komplementär sind.

34. Vorrichtung nach einem der Ansprüche 13 bis 33, **dadurch gekennzeichnet, dass** die M (M ≥ 1) Spannelemente (12) kraftschlüssig mit dem Körper (2) verbindbar sind.

35. Vorrichtung nach einem der Ansprüche 13 bis 33, **dadurch gekennzeichnet, dass** die M (M ≥ 1) Spannelemente (12) formschlüssig mit dem Körper (2) verbindbar sind.

36. Vorrichtung nach einem der Ansprüche 13 bis 35, **dadurch gekennzeichnet, dass** M = 1 und N = 1 ist.

37. Vorrichtung nach Anspruch 36, **dadurch gekennzeichnet, dass** das Spannelement (12) und der zweite Körper (2) einstückig sind.

38. Vorrichtung nach einem der Ansprüche 13 bis 37, **dadurch gekennzeichnet, dass** die äussere Mantelfläche (10) des Klemmelementes (8) und die Wandung des Hohlraumes (5) im Bereich von mindestens einem der Längenabschnittspaare Dᵢ und Eᵢ (i = 1 bis N) komplementär kreiszylindrisch ausgestaltet sind.

39. Vorrichtung nach einem der Ansprüche 13 bis 38, **dadurch gekennzeichnet, dass** K = 2, M = 2 und N = 2 ist.

40. Vorrichtung nach Anspruch 39, **dadurch gekennzeichnet, dass**
A) der Hohlraum (5) zwei Längenabschnitte (E;E') umfasst, wobei einer dieser Längenabschnitte (E;E') in die Oberseite (3) und der Andere in die Unterseite (4) mündet;
B) an die Bohrung (15) zwei Öffnungen (50;50') angrenzen, welche sich von je einem Ende (13;14) her auf einem Längenabschnitt (B;B') verjüngen;
C) das Klemmelement (8) von je einem Ende (13;14) her auf einem, den Längenabschnitt (B;B') beinhaltenden Längenabschnitt (A;A') senkrecht zur Längsachse (11) elastisch deformierbar ist, sowie auf je einem mindestens teilweise den Längenabschnitt (A;A') deckenden Längenabschnitt (D;D') eine zum Längenabschnitt (E;E') korrespondierende Form aufweist;
D) die Vorrichtung zwei Spannelemente (12;12') umfasst;
E) das Antriebselement (19) auf dem Längenabschnitt (F) ein Aussengewinde (22) und auf dem Längenabschnitt (F') ein Aussengewinde (51) umfasst, wobei eines der Gewinde ein Linksgewinde und das Andere ein Rechtsgewinde ist; und
F) eines der Spannelemente (12;12') ein zum Aussengewinde (22) korrespondierendes Innengewinde (24) und das Andere ein zum Aussengewinde (51) korrespondierendes Innengewinde (52) aufweist.

41. Vorrichtung nach Anspruch 40, **dadurch gekennzeichnet, dass** das Aussengewinde (22) und das Aussengewinde (51) Steigungswinkel mit gleichem Betrag aufweisen.

42. Vorrichtung nach Anspruch 40, **dadurch gekennzeichnet, dass** das Aussengewinde (22) und das Aussengewinde (51) Steigungswinkel mit ungleichem Betrag aufweisen.

43. Vorrichtung nach Anspruch 41 oder 42, **dadurch gekennzeichnet, dass** die Öffnungen (50;50') im Bereich der Längenabschnitt (B;B') konisch ausgebildet sind.

44. Vorrichtung nach Anspruch 41 oder 42, **dadurch gekennzeichnet, dass** die Spannelemente (12;12') im Bereich der Längenabschnitte (C;C') konisch ausgebildet sind.

45. Vorrichtung nach Anspruch 43, **dadurch gekennzeichnet, dass** die Spannelemente (12;12') im Bereich der Längenabschnitte (C;C') zu den Längenabschnitten (B;B') komplementär konisch ausgebildet sind.

46. Vorrichtung nach Anspruch 41 oder 42, **dadurch gekennzeichnet, dass** die Öffnungen (50;50') im Bereich der Längenabschnitte (B;B') keilförmig ausgebildet sind.

47. Vorrichtung nach Anspruch 41 oder 42, **dadurch gekennzeichnet, dass** die Spannelemente (12;12') im Bereich der Längenabschnitte (C;C') keilförmig ausgebildet sind.

48. Vorrichtung nach Anspruch 46, **dadurch gekennzeichnet, dass** die Spannelemente (12;12') im Bereich der Längenabschnitte (C;C') zu den Längenabschnitten (B;B') komplementär keilförmig ausgebildet sind.

49. Vorrichtung nach einem der Ansprüche 1 bis 48, **dadurch gekennzeichnet, dass** das Klemmelement (8) und der Körper (1) kraftschlüssig verbindbar sind.

50. Vorrichtung nach einem der Ansprüche 1 bis 48, **dadurch gekennzeichnet, dass** das Klemmelement (8) und der Körper (1) formschlüssig verbindbar sind.

51. Vorrichtung nach einem der Ansprüche 13 bis 50, **dadurch gekennzeichnet, dass** das Klemmelement (8) eine äussere Mantelfläche (10) umfasst, welche auf dem Längenabschnitt (Dᵢ) (i = 1 bis N) eine dreidimensionale Strukturierung aufweist.

52. Vorrichtung nach einem der Ansprüche 13 bis 51, **dadurch gekennzeichnet, dass** der Hohlraum (5) auf dem Längenabschnitt (Eᵢ) (i = 1 bis N) eine dreidimensionale Strukturierung aufweist.

53. Vorrichtung nach einem der Ansprüche 13 bis 50, **dadurch gekennzeichnet, dass** das Klemmelement (8) eine äussere Mantelfläche (10) umfasst, welche auf dem Längenabschnitt (Dᵢ) (i = 1 bis N) aufgerauht ist.

54. Vorrichtung nach einem der Ansprüche 13 bis 50, **dadurch gekennzeichnet, dass** der Hohlraum (5) auf dem Längenabschnitt (Eᵢ) (i = 1 bis N) aufgerauht ist.

55. Vorrichtung nach einem der Ansprüche 13 bis 50, **dadurch gekennzeichnet, dass** das Klemmelement (8) eine äussere Mantelfläche (10) umfasst, welche auf dem Längenabschnitt (Dᵢ) (i = 1 bis N) eine Verzahnung (33) aufweist.

56. Vorrichtung nach Anspruch 55, **dadurch gekennzeichnet, dass** der Hohlraum (5) auf dem Längenabschnitt (Eᵢ) (i = 1 bis N) eine zur Verzahnung (33) korrespondierende Verzahnung (35) aufweist.

57. Vorrichtung nach einem der Ansprüche 13 bis 56, **dadurch gekennzeichnet, dass** der Körper (1) im Längenabschnitt (Eᵢ) (i = 1 bis N) und das Klemmelement (8) im Längenabschnitt (Dᵢ) (i = 1 bis N) unterschiedliche Härten aufweisen.

## Claims

1. A device permitting a joint-like connection of two bodies, including
A) a first body (1) comprising a top surface (3), a bottom surface (4), and a cavity (5) extending coaxially to a central axis (6), the central axis (6) intersecting both the top surface (3) and the bottom surface (4), and the cavity (5) leading at least to the top surface (3);
B) a clamping member (8) with a longitudinal axis (11), a top end (13), a bottom end (14), and a bore (15) extending coaxially to the longitudinal axis (11) which leads to at least one aperture (50), the outside contour of the clamping member (8) in the area of the at least one aperture (50) being shaped in a form complementary to that of the cavity (5) and being resiliently deformable vertically to the longitudinal axis (11);
C) at least one tension member (12) insertable coaxially to the longitudinal axis (11) into the aperture (50), so that an axial displacement of the at least one tension member (12) within the corresponding aperture (50) may cause the clamping member (8) to be expanded and releasably locked within the cavity (5);
D) a second body (2) which is releasably connectable to the first body (1) by means of the at least one tension member (12) and of the clamping member (8);
E) a driving member (19) by means of which the at least one tension member (12) is axially displaceable relative to the corresponding aperture (50), whereby
F) the driving member (19) is connected to both the clamping member (8) and the at least one tension member (12) in such a way as to be axially in positive engagement while rotatable about the longitudinal axis (11),
whereby
G) the driving member (19) is connected to the clamping member (12) by means of a first threaded connection comprising an external screw thread (22) formed in a first longitudinal section (F) and a complementary, internal screw thread (24) formed in the tension member (12); and whereby
H) the driving member (19) is connected to the clamping member (8) by means of a second opposedly threaded connection comprising an external screw thread (30) formed in a longitudinal section (G) and a complementary, internal screw thread (31) formed in the bore (15) of the clamping member (8).

2. A device as claimed in claim 1, **characterised in that** the clamping member (8) has a through bore extending along the longitudinal axis (11).

3. A device as claimed in claim 1 or 2, **characterised in that** the clamping member (8) comprises X slots (47) extending substantially in an axial direction and connecting the aperture (50) with the outside contour of the clamping member (8).

4. A device as claimed in claim 3, **characterised in that** X = 1.

5. A device as claimed in claim 3, **characterised in that** X = 2 and that the two slots (47) are arranged collaterally opposite to each other.

6. A device as claimed in claim 3, **characterised in that** X < 12 and X ≥ 3, X being preferably = 6.

7. A device as claimed in claim 5, **characterised in that** the two slots (47) and the respective aperture (50) continuously merge into each other.

8. A device as claimed in any of the claims 3 to 7, **characterised in that** the at least one aperture (50) extends coaxially to the longitudinal axis (11).

9. A device as claimed in claim 8, **characterised in that** the at least one aperture (50) is rotationally symmetrical.

10. A device as claimed in any of the claims 1 to 9, **characterised in that** the longitudinal axis (11) and the central axis (6) are in alignment.

11. A device as claimed in any of the claims 1 to 10, **characterised in that** the outside contour of the clamping member (8) in the area of contact with the wall defining the cavity (5) is rotationally symmetrical relative to the longitudinal axis (11).

12. A device as claimed in any of the claims 1 to 11, **characterised in that** it comprises at least one tension member (12), said at least one tension member (12) being displaceable in both directions coaxially to the longitudinal axis (11) and relative to the at least one aperture (50) by means of the driving member (19).

13. A device as claimed in any of the claims 1 to 12, **characterised in that**
A) the wall of the cavity (5) includes N (N ≥ 1) longitudinal sections (Eᵢ) (i = 1 to N);
B) the clamping member (8) has at least K (K ≥ 1) longitudinal sections (Aᵢ) (i = 1 to K) extending axially which are resiliently deformable in a direction vertical to the longitudinal axis (11);
C) the outside contour of the clamping member (8) in the area of the longitudinal sections (Aᵢ) (i = 1 to K) has at least N (N ≥ 1) longitudinal sections (Dᵢ) (i = 1 to N) extending axially which are complementary, as far as their geometry and their surface structure are concerned, to the longitudinal sections (Eᵢ) (i = 1 to N);
D) the device comprises M (M ≥ 1) tension members (12) having each a top end (16) and a bottom end (17);
E) the at least one aperture (50) of the clamping member (8) has in total at least M (M ≥ 1) axially arranged longitudinal sections (Bᵢ) (i = 1 to M) which enter into contact with the M (M ≥ 1) tension members (12), as they are spread apart;
F) the M (M ≥ 1) tension members (12) are actively displaceable in both directions coaxially to the at least one aperture (50) by means of the driving member (19), whereby the resilient longitudinal sections (Aᵢ) (i = 1 to K) are spread apart or moved back to their initial positions vertically to the longitudinal axis (11) so that the longitudinal sections (Dᵢ) (i = 1 to N) may be locked within, or inversely released from, the longitudinal sections (Eᵢ) (i = 1 to N) of the cavity (5),
G) the driving member (19) being connected in a longitudinal section (G) extending coaxially to the longitudinal axis (11) to the clamping member (8) and in M (M ≥ 1) longitudinal sections (Fⱼ) (j = 1 to M) extending coaxially to the longitudinal axis (11) to the M (M ≥ 1) tension members (12) in such a way as to be axially in positive engagement while rotatable about the longitudinal axis (11).

14. A device as claimed in claim 12, **characterised in that** the aperture (50) has an axially tapered form extending at least over the area of the longitudinal sections (Dᵢ) (i = 1 to N).

15. A device as claimed in claim 13, **characterised in that** the tension members (12) have an axially tapered form extending at least over the area of the longitudinal sections (Cᵢ) (i = 1 to M).

16. A device as claimed in claim 14, **characterised in that** the tension members (12) have an axially tapered form extending over the area of the longitudinal sections (Cᵢ) (i = 1 to M) and being complementary to the longitudinal sections (Dᵢ) (i = 1 to N).

17. A device as claimed in any of the claims 13 to 16, **characterised in that** K = N.

18. A device as claimed in any of the claims 13 to 17, **characterised in that** K = M.

19. A device as claimed in any of the claims 13 to 18, **characterised in that** M = N.

20. A device as claimed in any of the claims 13 to 19, **characterised in that** K = 1.

21. A device as claimed in any of the claims 13 to 20, **characterised in that** M = 1.

22. A device as claimed in any of the claims 13 to 21, **characterised in that** N = 1.

23. A device as claimed in claims 20 to 22, **characterised in that** the tension member (12) has a bore (23) extending concentrically to the longitudinal axis (11) and that in the longitudinal section (Fᵢ) (i = 1) the driving member (19) may be screwed into said bore (23) coaxially to the longitudinal axis (11) while being connected, in the longitudinal section (G), to the clamping member (8) by means of anchoring means (29) in such a way as to be axially in positive engagement while rotatable about the longitudinal axis (11).

24. A device as claimed in claim 23, **characterised in that** the pitch angles of the two threaded connections are identical.

25. A device as claimed in claim 22 or 23, **characterised in that** the pitch angles of the two threaded connections are different.

26. A device as claimed in any of the claims 13 to 35, **characterised in that** the clamping member (8) in the area of the longitudinal sections (Eᵢ) (i = 1 to N) of the cavity (5) is connectable to the first body (1) in such a way as to be in non-positive engagement.

27. A device as claimed in any of the claims 13 to 25, **characterised in that** the clamping member (8) in the area of the longitudinal sections (Eᵢ) (i = 1 to N) of the cavity (5) is connectable to the first body (1) in such a way as to be in positive engagement.

28. A device as claimed in any of the claims 13 to 27, **characterised in that** the M (M ≥ 1) tension members (12) in the longitudinal sections (Cᵢ) (i = 1 to M) have a conical taper.

29. A device as claimed in any of the claims 13 to 27, **characterised in that** the clamping member (8) in the longitudinal sections (Bᵢ) (i = 1 to M) has a conical taper.

30. A device as claimed in claims 28 and 29, **characterised in that** said tapers are complementary.

31. A device as claimed in any of the claims 13 to 27, **characterised in that** the M (M > 1) tension members (12) in the longitudinal sections (Cᵢ) (i = 1 to M) have a wedge-shaped taper.

32. A device as claimed in any of the claims 13 to 27, **characterised in that** the clamping member (8) in the longitudinal sections (Bᵢ) (i = 1 to M) has a wedge-shaped taper.

33. A device as claimed in claims 31 and 32, **characterised in that** said tapers are complementary.

34. A device as claimed in any of the claims 13 to 33, **characterised in that** the M (M ≥ 1) tension members (12) are connectable to the body (2) in such a way as to be in non-positive engagement.

35. A device as claimed in any of the claims 13 to 33, **characterised in that** the M (M ≥ 1) tension members (12) are connectable to the body (2) in such a way as to be in positive engagement.

36. A device as claimed in any of the claims 13 to 35, **characterised in that** M = 1 and N = 1.

37. A device as claimed in claim 36, **characterised in that** the tension member (12) is integral with the second body (2).

38. A device as claimed in any of the claims 13 to 37, **characterised in that** the outer lateral area (10) of the clamping member (8) and the wall of the cavity (5) in the area of at least one of the pairs of longitudinal sections Dᵢ and Eᵢ (i = 1 to N) are shaped in complementary, circularly cylindrical forms.

39. A device as claimed in any of the claims 13 to 38, **characterised in that** K = 2, M = 2 and N = 2.

40. A device as claimed in claim 49, **characterised in that**
A) the cavity (5) comprises two longitudinal sections (E,E'), one of said longitudinal sections (E,E') leading to the top surface (3), the other to the bottom surface (4);
B) adjacent to the bore (15), two apertures (50;50') are arranged which have tapered forms, beginning each from one end (13;14) and extending over a longitudinal section (B;B');
C) the clamping member (8) in a longitudinal section (A;A') beginning from the end (13;14) and including the longitudinal section (B;B') is resiliently deformable vertically to the longitudinal axis (11) and has on its longitudinal section (D;D'), which at least partially includes the longitudinal section (A;A'), a form corresponding to that of the longitudinal section (E;E');
D) the device comprises two tension members (12;12');
E) the driving member (19) comprises an external screw thread (22) formed in the longitudinal section (F) and an external screw thread (51) formed in the longitudinal section (F'), one of said screw threads being a left-hand thread, the other a right-hand thread; and
F) one of the tension members (12;12') has an internal screw thread (24) corresponding to the external screw thread (22) and the other has an internal screw thread (52) corresponding to the external screw thread (51).

41. A device as claimed in claim 40, **characterised in that** the external screw thread (22) and the external screw thread (51) have pitch angles of identical amounts.

42. A device as claimed in claim 40, **characterised in that** the external screw thread (22) and the external screw thread (51) have pitch angles of different amounts.

43. A device as claimed in claim 41 or 42, **characterised in that** the apertures (50;50') in the areas of the longitudinal sections (B;B') are conically shaped.

44. A device as claimed in claim 41 or 42, **characterised in that** the tension members (12;12') in the areas of the longitudinal sections (C;C') are conically shaped.

45. A device as claimed in claim 43, **characterised in that** the tension members (12;12') in the areas of the longitudinal sections (C;C') are conically complementary to the longitudinal sections (B;B').

46. A device as claimed in claim 41 or 42, **characterised in that** the apertures (50;50') in the areas of the longitudinal sections (B;B') are wedge-shaped.

47. A device as claimed in claim 41 or 42, **characterised in that** the tension members (12;12') in the areas of the longitudinal sections (C;C') are wedge-shaped.

48. A device as claimed in claim 46, **characterised in that** the tension members (12;12') in the areas of the longitudinal sections (C;C') are wedge-shaped and complementary to the longitudinal sections (B;B').

49. A device as claimed in any of the claims 1 to 48, **characterised in that** the clamping member (8) and the body (1) are connectable in such a way as to be in non-positive engagement.

50. A device as claimed in any of the claims 1 to 48, **characterised in that** the clamping member (8) and the body (1) are connectable in such a way as to be in positive engagement.

51. A device as claimed in any of the claims 13 to 50, **characterised in that** the clamping member (8) comprises an outer lateral surface (10) which in the longitudinal section (Dᵢ) (i = 1 to N) is provided with a three-dimensionally structured portion.

52. A device as claimed in any of the claims 13 to 51, **characterised in that** the cavity (5) in the longitudinal section (Eᵢ) (i = 1 to N) is provided with a three-dimensionally structured portion.

53. A device as claimed in any of the claims 13 to 50, **characterised in that** the clamping member (8) comprises an outer lateral surface (10) which is roughened in the longitudinal section (Dᵢ) (i = 1 to N).

54. A device as claimed in any of the claims 13 to 50, **characterised in that** the cavity (5) is roughened in the longitudinal section (E;) (i = 1 to N).

55. A device as claimed in any of the claims 13 to 50, **characterised in that** the clamping member (8) comprises an outer lateral surface (10) which in the longitudinal section (Dᵢ) (i = 1 to N) is provided with a toothing (33).

56. A device as claimed in claim 55, **characterised in that** the cavity (5) in the longitudinal section (Eᵢ) (i = 1 to N) is provided with a toothing (35) which corresponds to the toothing (33).

57. A device as claimed in any of the claims 13 to 56, **characterised in that** the body (1) in the longitudinal section (Eᵢ) (i = 1 to N) and the clamping member (8) in the longitudinal section (Dᵢ) (i = 1 to N) have different degrees of hardness.

## Revendications

1. Dispositif pour relier deux corps de manière articulée, comprenant
A) un premier corps (1), lequel comprend une face supérieure (3), une face inférieure (4) et un espace creux (5) coaxial à un axe central (6), dans lequel l'axe central (6) coupe la face supérieure (3) et la face inférieure (4) et l'espace creux (5) débouche au moins dans la face supérieure (3) ;
B) un élément de serrage (8) avec un axe longitudinal (11), une extrémité supérieure (13), une extrémité inférieure (14) et un alésage (15) coaxial à l'axe longitudinal (11), lequel débouche dans au moins une ouverture (50), dans lequel le profil extérieur de l'élément de serrage (8) dans la zone de la au moins une ouverture (50) est conçu de manière complémentaire à l'espace creux (5) et pour être déformable de manière élastique perpendiculairement à l'axe longitudinal (11) ;
C) au moins un élément de blocage (12) pouvant être inséré coaxialement à l'axe longitudinal (11) dans l'ouverture (50), de sorte que lors d'un déplacement axial du au moins un élément de blocage (12) dans l'ouverture (50) correspondante, l'élément de serrage (8) peut se dilater et être bloqué de manière amovible dans l'espace creux (5) ;
D) un deuxième corps (2), lequel peut être relié de manière amovible au premier corps (1) au moyen du au moins un élément de blocage (12) et de l'élément de serrage (8) ;
E) un élément d'entraînement (19), au moyen duquel le au moins un élément de blocage (12) peut être déplacé axialement par rapport à l'ouverture (50) correspondante, dans lequel
F) l'élément d'entraînement (19) est relié à la fois à l'élément de serrage (8) et au moins un élément de blocage (12) de manière à pouvoir tourner autour de l'axe longitudinal (11) et à s'assembler avec eux par emboîtement dans l'axe, dans lequel
G) l'élément d'entraînement (19) est relié à l'élément de blocage (12) au moyen d'un premier assemblage fileté, lequel comprend un filetage (22) sur un premier segment (F) et un taraudage (24) complémentaire à celui-ci dans l'élément de blocage (12) ; et dans lequel
H) l'élément d'entraînement (19) est relié à élément de serrage (8) au moyen d'un deuxième assemblage fileté en sens contraire, lequel comprend un filetage (30) sur un deuxième segment (G) et un taraudage (31) complémentaire à celui-ci dans l'alésage (15) de l'élément de serrage (8).

2. Dispositif selon la revendication 1, **caractérisé en ce que** l'élément de serrage (8) présente une ouverture continue le long de l'axe longitudinal (11).

3. Dispositif selon la revendication 1 ou 2, **caractérisé en ce que** l'élément de serrage (8) comprend X fentes (47), lesquelles s'étendent essentiellement axialement et relient l'ouverture (50) au profil extérieur de l'élément de serrage (8).

4. Dispositif selon la revendication 3, **caractérisé en ce que** X = 1.

5. Dispositif selon la revendication 3, **caractérisé en ce que** X = 2 et les deux fentes (47) sont opposées collatéralement.

6. Dispositif selon la revendication 3, **caractérisé en ce que** X ≤ 12 et X ≥ 3, de préférence X = 6.

7. Dispositif selon la revendication 5, **caractérisé en ce que** la transition entre les deux fentes (47) et l'ouverture (50) correspondante est sans aspérités.

8. Dispositif selon l'une quelconque des revendications 3 à 7, **caractérisé en ce que** la au moins une ouverture (50) est située coaxialement à l'axe longitudinal (11).

9. Dispositif selon la revendication 8, **caractérisé en ce que** la au moins une ouverture (50) est à symétrie de rotation.

10. Dispositif selon l'une quelconque des revendications 1 à 9, **caractérisé en ce que** l'axe longitudinal (11) et l'axe central (6) sont alignés.

11. Dispositif selon l'une quelconque des revendications 1 à 10, **caractérisé en ce que** le profil extérieur de l'élément de serrage (8) est à symétrie de rotation par rapport à l'axe longitudinal (11) au moins dans la zone de contact avec la paroi délimitant l'espace creux (5).

12. Dispositif selon l'une quelconque des revendications 1 à 11, **caractérisé en ce qu'**il comprend au moins un élément de blocage (12), dans lequel ce au moins un élément de blocage (12) peut être déplacé au moyen de l'élément d'entraînement (19) coaxialement à l'axe longitudinal (11) et dans les deux sens par rapport à la au moins une ouverture (50).

13. Dispositif selon l'une quelconque des revendications 1 à 12,
**caractérisé en ce que**
A) la paroi de l'espace creux (5) comprend axialement entre autres N (N ≥ 1) segments (Eᵢ) (i = 1 à N) à symétrie de rotation par rapport à l'axe central (6) ;
B) l'élément de serrage (8) présente axialement au moins K (K ≥ 1) segments (Aᵢ) (i = 1 à K), lesquels sont déformables de manière élastique perpendiculairement à l'axe longitudinal (11) ;
C) le profil extérieur de l'élément de serrage (8), dans la zone des segments (Aᵢ) (i = 1 à K), présente axialement au moins N (N ≥ 1) segments (Dᵢ) (i = 1 à N) complémentaires, en termes de géométrie et de texture de surface, aux segments (Eᵢ) (i = 1 à N) de l'espace creux (5) ;
D) le dispositif M (M ≥ 1) comprend des éléments de blocage (12) ayant chacun une extrémité supérieure (16) et une extrémité inférieure (17) ;
E) la au moins une ouverture (50) de l'élément de serrage (8) présente axialement au total au moins M (M ≥ 1) segments (Bi) (i = 1 à M) venant en contact avec les M (M ≥ 1) éléments de blocage (12) lors de l'écartement ;
F) les M (M ≥ 1) éléments de blocage (12) peuvent être déplacés activement, au moyen de l'élément d'entraînement (19), coaxialement dans la au moins une ouverture (50) dans les deux sens et les segments élastiques (Aᵢ) (i = 1 à K) peuvent ainsi être écartés ou rapprochés perpendiculairement à l'axe longitudinal (11) et les segments (Dᵢ) (i = 1 à N) peuvent alors être bloqués ou débloqués dans les segments (Eᵢ) (i = 1 à N) de l'espace creux (5) ; dans lequel
G) l'élément d'entraînement (19) peut tourner autour de l'axe longitudinal (11) au niveau d'un segment (G) coaxialement à l'axe longitudinal (11), conjointement avec l'élément de serrage (8) et au niveau des M (M ≥ 1) segments (Fⱼ) (j = 1 à M) coaxialement à l'axe longitudinal (11), conjointement avec les M (M ≥ 1) éléments de blocage (12) et il est relié axialement par emboîtement à ces éléments.

14. Dispositif selon la revendication 13, **caractérisé en ce que** la au moins une ouverture (50) est rétrécie axialement au moins dans la zone des segments (Dᵢ) (i = 1 à N).

15. Dispositif selon la revendication 13, **caractérisé en ce que** les éléments de blocage (12) sont rétrécis axialement au moins dans la zone des segments (Cᵢ) (i = 1 à M).

16. Dispositif selon la revendication 14, **caractérisé en ce que** les éléments de blocage (12) sont rétrécis axialement dans la zone des segments (Cᵢ) (i = 1 à M) complémentairement aux segments (Dᵢ) (i = 1 à N).

17. Dispositif selon l'une quelconque des revendications 13 à 16, **caractérisé en ce que** K = N.

18. Dispositif selon l'une quelconque des revendications 13 à 17, **caractérisé en ce que** K = M.

19. Dispositif selon l'une quelconque des revendications 13 à 18, **caractérisé en ce que** M = N.

20. Dispositif selon l'une quelconque des revendications 13 à 19, **caractérisé en ce que** K = 1.

21. Dispositif selon l'une quelconque des revendications 13 à 20, **caractérisé en ce que** M = 1.

22. Dispositif selon l'une quelconque des revendications 13 à 21, **caractérisé en ce que** N = 1.

23. Dispositif selon les revendications 20 à 22, **caractérisé en ce que** l'élément de blocage (12) présente, concentriquement à l'axe longitudinal (11), un alésage (23), et l'élément d'entraînement (19) peut être vissé, au niveau du segment (Fᵢ) (i = 1) coaxialement à l'axe longitudinal (11), dans l'alésage (23) et peut tourner autour de l'axe longitudinal (11) au niveau du segment (G), à l'aide de moyens d'ancrage (2) conjointement avec l'élément de serrage (8) et il est relié axialement par emboîtement à cet élément.

24. Dispositif selon la revendication 23, **caractérisé en ce que** l'angle de pas des deux assemblages filetés est identique.

25. Dispositif selon la revendication 22 ou 23, **caractérisé en ce que** l'angle de pas des deux assemblages filetés est différent.

26. Dispositif selon l'une quelconque des revendications 13 à 25, **caractérisé en ce que** l'élément de serrage (8) dans la zone des segments (Eᵢ) (i = 1 à N) de l'espace creux (5) peut être relié au premier corps (1) par enfoncement forcé.

27. Dispositif selon l'une quelconque des revendications 13 à 25, **caractérisé en ce que** l'élément de serrage (8) dans la zone des segments (Eᵢ) (i = 1 à N) de l'espace creux (5) peut être relié au premier corps (1) par emboîtement.

28. Dispositif selon l'une quelconque des revendications 13 à 27, **caractérisé en ce que** les M (M ≥1) éléments de blocage (12) sont rétrécis de manière conique au niveau des segments (Cᵢ) (i = 1 à M).

29. Dispositif selon l'une quelconque des revendications 13 à 27, **caractérisé en ce que** l'élément de serrage (8) est rétréci de manière conique au niveau des segments (Bᵢ) (i = 1 à M).

30. Dispositif selon les revendications 28 et 29, **caractérisé en ce que** les rétrécissements sont complémentaires.

31. Dispositif selon l'une quelconque des revendications 13 à 27, **caractérisé en ce que** les M (M ≥ 1) éléments de blocage (12) sont rétrécis de manière cunéiforme au niveau des segments (Cᵢ) (i = 1 à M).

32. Dispositif selon l'une quelconque des revendications 13 à 27, **caractérisé en ce que** l'élément de serrage (8) est rétréci de manière cunéiforme au niveau des segments (Bᵢ) (i = 1 à M).

33. Dispositif selon les revendications 31 et 32, **caractérisé en ce que** les rétrécissements sont complémentaires.

34. Dispositif selon l'une quelconque des revendications 13 à 33, **caractérisé en ce que** les M (M ≥ 1) éléments de blocage (12) peuvent être reliés au corps (2) par enfoncement forcé.

35. Dispositif selon l'une quelconque des revendications 13 à 33, **caractérisé en ce que** les M (M ≥ 1) éléments de blocage (12) peuvent être reliés au corps (2) par emboîtement.

36. Dispositif selon l'une quelconque des revendications 13 à 35, **caractérisé en ce que** M = 1 et N = 1.

37. Dispositif selon la revendication 36, **caractérisé en ce que** l'élément de blocage (12) et le deuxième corps (2) sont formés d'une seule pièce.

38. Dispositif selon l'une quelconque des revendications 13 à 37, **caractérisé en ce que** l'enveloppe externe (10) de l'élément de serrage (8) et la paroi de l'espace creux (5) dans la zone d'au moins une paire de segments Dᵢ et Eᵢ (i = 1 à N) sont conçus sous la forme de cylindres creux complémentaires.

39. Dispositif selon l'une quelconque des revendications 13 à 38, **caractérisé en ce que** K = 2, M = 2 et N = 2.

40. Dispositif selon la revendication **caractérisé en ce que**
A) l'espace creux (5) comprend deux segments (E ; E'), dans lequel l'un de ces segments (E ; E') débouche dans la face supérieure (3) et l'autre dans la face inférieure (4) ;
B) deux ouvertures (50 ; 50') sont adjacentes à l'alésage (15), lesquelles sont chacune rétrécies au niveau d'un segment (B ; B') en partant d'une extrémité (13 ; 14) ;
C) l'élément de serrage (8), en partant de chaque extrémité (13 ; 14), peut être déformé de manière élastique au niveau d'un segment (A ; A') contenant le segment (B ; B') perpendiculairement à l'axe longitudinal (11), et présente, au niveau de chacun des segments (D ; D') couvrant au moins en partie les segments (A ; A'), une forme correspondant à celle du segment (E ; E') ;
D) le dispositif comprend deux éléments de blocage (12 ; 12') ;
E) l'élément d'entraînement (19) comprend un filetage (22) au niveau du segment (F) et un taraudage (51) au niveau du segment (F'), dans lequel l'un des filets est un filet gauche et l'autre un filet droit ; et
F) l'un des éléments de blocage (12 ; 12') présente un taraudage (24) correspondant au filetage (22) et l'autre un taraudage (52) correspondant au filetage (51).

41. Dispositif selon la revendication 40, **caractérisé en ce que** le filetage (22) et le filetage (51) présentent un angle de pas de même valeur.

42. Dispositif selon la revendication 40, **caractérisé en ce que** le filetage (22) et le filetage (51) présentent un angle de pas de valeur différente.

43. Dispositif selon la revendication 41 ou 42, **caractérisé en ce que** les ouvertures (50 ; 50') sont réalisées sous forme conique dans la zone des segments (B ; B').

44. Dispositif selon la revendication 41 ou 42, **caractérisé en ce que** les éléments de blocage (12 ; 12') sont réalisés sous forme conique dans la zone des segments (C ; C').

45. Dispositif selon la revendication 43, **caractérisé en ce que** les éléments de blocage (12 ; 12') sont réalisés sous une forme conique complémentaire à celle des segments (B ; B') dans la zone des segments (C ; C').

46. Dispositif selon la revendication 41 ou 42, **caractérisé en ce que** les ouvertures (50 ; 50') sont réalisées sous forme de coin dans la zone des segments (B ; B').

47. Dispositif selon la revendication 41 ou 42, **caractérisé en ce que** les éléments de blocage (12 ; 12') sont réalisés sous forme de coin dans la zone des segments (C ; C').

48. Dispositif selon la revendication 46, **caractérisé en ce que** les éléments de blocage (12 ; 12') sont réalisés sous une forme de coin complémentaire à celle des segments (B ; B') dans la zone des segments (C ; C').

49. Dispositif selon l'une quelconque des revendications 1 à 48, **caractérisé en ce que** l'élément de serrage (8) et le corps (1) peuvent être assemblés par enfoncement forcé.

50. Dispositif selon l'une quelconque des revendications 1 à 48, **caractérisé en ce que** l'élément de serrage (8) et le corps (1) peuvent être assemblés par emboîtement.

51. Dispositif selon l'une quelconque des revendications 13 à 50, **caractérisé en ce que** l'élément de serrage (8) comprend une enveloppe externe (10), laquelle présente une texture tridimensionnelle au niveau du segment (Dᵢ) (i = 1 à N).

52. Dispositif selon l'une quelconque des revendications 13 à 51, **caractérisé en ce que** l'espace creux (5) présente une texture tridimensionnelle au niveau du segment (Eᵢ) (i = 1 à N).

53. Dispositif selon l'une quelconque des revendications 13 à 50, **caractérisé en ce que** l'élément de serrage (8) comprend une enveloppe externe (10), laquelle a été rendue rugueuse au niveau du segment (Dᵢ) (i = 1 à N).

54. Dispositif selon l'une quelconque des revendications 13 à 50, **caractérisé en ce que** l'espace creux (5) a été rendu rugueux au niveau du segment (Eᵢ) (i = 1 à N).

55. Dispositif selon l'une quelconque des revendications 13 à 50, **caractérisé en ce que** l'élément de serrage (8) comprend une enveloppe externe (10), laquelle présente une denture (33) au niveau du segment (Dᵢ) (i = 1 à N).

56. Dispositif selon la revendication 55, **caractérisé en ce que** l'espace creux (5) présente une denture (35) correspondant à la denture (33) au niveau du segment (Eᵢ) (i = 1 à N).

57. Dispositif selon l'une quelconque des revendications 13 à 56, **caractérisé en ce que** le corps (1) dans le segment (Eᵢ) (i = 1 à N) et l'élément de serrage (8) dans le segment (Dᵢ) (i = 1 à N) présentent différentes duretés.
